(19) Europäisches Patentamt
European Patent Office
Office européen des brevets

(11) Numéro de publication: **0 345 100 A1**

(12) **DEMANDE DE BREVET EUROPEEN**

(21) Numéro de dépôt: **89401054.5**

(22) Date de dépôt: **17.04.89**

(51) Int. Cl.⁴: **G 01 N 33/92**

(30) Priorité: **18.04.88 FR 8805071**

(43) Date de publication de la demande:
**06.12.89 Bulletin 89/49**

(84) Etats contractants désignés:
**AT BE CH DE ES FR GB GR IT LI LU NL SE**

(71) Demandeur: **INSTITUT PASTEUR**
**28, rue du Docteur Roux**
**F-75724 Paris Cédex 15 (FR)**

**INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur: **Dray, Fernand**
**4 Rue Guynemer**
**F-75006 Paris (FR)**

**Tiberghien, Christophe**
**14 Rue Georges Bizet**
**F-78370 Plaisir (FR)**

**Junier, Marie-Pierre**
**1 Rue des Morillons**
**F-75015 Paris (FR)**

(74) Mandataire: **Ores, Irène et al**
**CABINET ORES 6, Avenue de Messine**
**F-75008 Paris (FR)**

Le titre de l'invention a été modifié (Directives relatives à l'examen pratiqué à l'OEB, A-III, 7.3)

(54) **Préparation de récepteurs du PAF et méthode de dosage du PAF.**

(57) Préparation de récepteur du PAF constituée par l'association d'une préparation membranaire de récepteur du PAF avec des agents de protection appropriés, tels que des inhibiteurs de protéases par exemple.

Méthode de dosage du PAF présent dans un échantillon biologique, dans laquelle on met en compétition un PAF marqué introduit dans le milieu de dosage, avec le PAF non marqué contenu dans l'échantillon biologique, pour leur occupation des sites récepteurs du PAF présents dans une préparation de récepteur du PAF également introduite dans le milieu de dosage.

Applications au diagnostic de différents troubles.

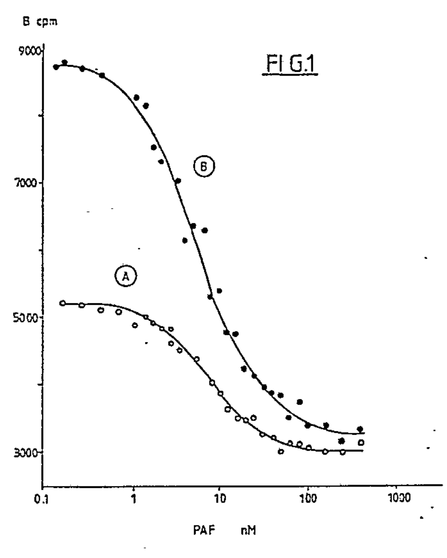


FIG.1

EP 0 345 100 A1

**Description**

**NOUVELLE PREPARATION DE RECEPTEURS DU PAF ET NOUVELLE METHODE DE DOSAGE DU PAF**

La présente invention a pour objet une nouvelle méthode de dosage du "platelet-activating factor" ou PAF.

Le "platelet-activating factor" ou PAF est l'un des plus puissants médiateurs chimiques de nature lipidique actuellement identifiés ; il est impliqué dans une variété de conditions physiopathologiques (thrombose artérielle, inflammation aigüe, choc endotoxique, réactions allergiques aigües, ovoimplantation), ce qui explique l'importance attachée par les Chercheurs à la mise au point d'un test de détermination sensible, spécifique de ce puissant médiateur.

La caractérisation du PAF et son obtention ont été décrites pour la première fois par BENVENISTE et al. [J. EXP. MED., 136 (1972), pp. 1356], puis sa présence dans les leucocytes humains [BENVENISTE, NATURE, 249 (1974), pp. 581], ses actions d'agrégation et de sécrétion sur les plaquettes humaines, et la plupart de ses caractéristiques physicochimiques, y compris sa nature phospholipidique, ont ensuite été démontrées [BENVENISTE et al., LANCET, 1 (1975), pp. 344 ; BENVENISTE et al., NATURE, 269 (1977), pp. 170] La poursuite des travaux sur le PAF a permis de mettre en évidence que celui-ci est un éthérophospholipide dont la caractérisation chimique a permis d'établir sa structure comme étant celle d'une 1-0-alkyl-2-acétyl-sn-glycérol-3-phosphorylcholine qui répond à la formule ci-après:

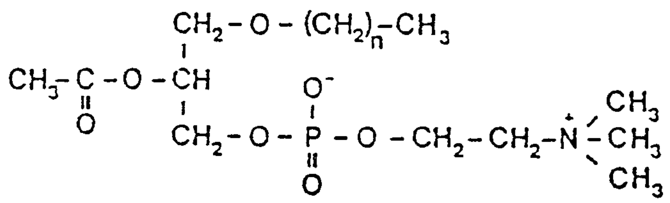

où n est égal à 15 ou 17. [BENVENISTE et al., CR Acad. Sci. (III) 289, page 1017 (1979) ; DEMOPOULOS et al., J. BIOL. CHEM., 254, page 9355 (1979)].

Son implication dans les processus inflammatoires et allergiques a été démontrée et il joue un rôle dans le système immunitaire et sur le système nerveux central.

La détection de cette molécule est difficile car les quantités de PAF produites dans les divers systèmes biologiques étudiés sont de l'ordre du nanogramme ou du fentogramme et requièrent des systèmes de dosage sensibles.

Cependant la compréhension du rôle du PAF aussi bien dans les processus physiologiques normaux que dans les états pathologiques requiert que l'on puisse détecter et mesurer ce puissant médiateur même à de très faibles concentrations.

Plusieurs méthodes de dosage ont été proposées à cet effet : la réalisation de tests basés sur les propriétés du PAF de stimulateur de l'agrégation de plaquettes humaines ou lapines. Bien que de telles méthodes soient sensibles, leur mise en oeuvre est difficile. En outre, les méthodes basées sur l'agrégation plaquettaire présentent l'inconvénient d'une variabilité qui est inhérente à leur qualité de tests biologiques et d'un manque de fiabilité dû au fait que d'autres substances que le PAF induisent également l'agrégation plaquettaire.

Une autre méthode de dosage biologique a été proposée : basée sur la propriété du PAF de stimulateur de la libération des granules de sécrétion plaquettaires, elle consiste à doser les taux de libération des substances générées par les plaquettes, notamment la sérotonine, dont la libération peut être quantifiée si les plaquettes sont préincubées en présence de sérotonine marquée. Les inconvénients de cette méthode sont les mêmes que ceux de la méthode qui utilise l'agrégation plaquettaire.

Ces inconvénients, auxquels s'ajoute le fait que les dosages biologiques ont lieu en temps réel en sorte qu'ils ne permettent le dosage que d'un nombre limité d'échantillons, ont orienté les Chercheurs vers la mise au point de dosages immunologiques, et en particulier de dosages radioimmunologiques du PAF.

NISHIHIRA et al. [J. BIOCHEM., 95 (1984), pages 1247-1251] ont ainsi décrit la production d'anticorps polyclonaux spécifiques anti-PAF et leur caractérisation par des tests d'agglutination, de double-immunodiffusion, d'inhibition de l'agrégation plaquettaire et de radioimmunoprécipitation, puis KARASAWA ET AL. [J. BIOCHEM., 102, 451-453 (1987)] ont décrit la préparation d'anticorps polyclonaux anti-PAF spécifiques, par immunisation de lapins à l'aide d'un conjugué haptène-BSA, l'haptène étant constitué par un dérivé synthétique du PAF, résistant à l'égard de l'inactivation enzymatique par le plasma ou des tissus et qui peut se lier à la BSA par covalence. L'activité des anticorps a été déterminée par la technique ELISA ; mais le test d'inhibition par la technique ELISA qui visait à mettre en évidence que ces anticorps n'ont pas de réaction croisée avec les anticorps du PAF, introduit une incertitude quant à certains dérivés du PAF. La sensibilité de ces dosages immunologiques s'est avérée moins grande que celle des dosages biologiques.

Parallèlement au développement des dosages immunologiques, le champ des connaissances concernant les mécanismes d'action des hormones s'est étendu et il s'est avéré que l'action de la plupart des hormones s'exerce par l'intermédiaire de leur liaison à un site récepteur hormone-spécifique intracellulaire ou situé sur la membrane des cellules-cibles et entraînant l'activation d'effecteurs couplés au complexe hormone-récepteur. Les différents récepteurs étudiés présentent des constantes d'association élevées et une haute spécificité.

En 1968 fut publiée une méthode de dosage d'hormones utilisant un récepteur cellulaire [KORENMAN, J. CLIN. ENDOCRINOL. METAB. 28, pp. 127, 1968], puis LEFKOWITZ et al. [SCIENCE, 170, pp. 633, 1970] ont décrit l'utilisation d'un récepteur de membrane cellulaire couplé à un système adénylcyclase, pour le dosage d'hormones et GILMAN, d'une part [PROC. NATL. ACAD. SCI. USA, 67, pp. 305, 1970] et WALTON et al., d'autre part, [BIOCHEMISTRY, 9, pp. 4223, 1970] ont décrit des méthodes de mesure de cAMP en utilisant un récepteur intracellulaire.

Les avantages de l'utilisation de récepteurs cellulaires dans un dosage sont nombreux :
- haute affinité du système récepteur (conférant une bonne sensibilité au dosage développé),
- haute spécificité,
- données nombreuses sur le système utilisé, sur la spécificité du modèle et sur les caractéristiques biochimiques,
- origines multiples du récepteur : le récepteur peut provenir d'espèces différentes ou de tissus différents et présenter des caractéristiques communes, contrairement aux caractéristiques des anticorps utilisés dans un dosage immunologique, qui n'auront pas de propriétés similaires s'ils proviennent d'origines différentes.

Les dosages d'hormones polypeptidiques à l'aide de radio-récepteurs font application du principe de la compétition entre l'hormone marquée ($^{125}I$ marquée, par exemple), et l'hormone non marquée pour leur fixation à des récepteurs spécifiques de l'hormone considérée. Il semblerait selon les Chercheurs qui ont décrit ces dosages [LEFKOWITZ et al., SCIENCE, Vol. 170, pages 633-635, 6 Novembre 1970] que ceux-ci présentent les avantages des immunoessais tout en présentant la spécificité des méthodes biologiques.

HWANG et al. [BIOCHEMISTRY, 22 (1983), pages 4756-4763] ont identifié, en utilisant du PAF marqué par du tritium, ses sites de liaison spécifiques sur des membranes de plaquettes de lapin ; ils ont évalué le nombre des sites de liaison à 1,61 (+ 0,34)x$10^{12}$/mg de membrane, ce qui correspond à environ 150-300 récepteurs/plaquette. Une liaison spécifique du PAF a été démontrée en utilisant des préparations membranaires purifiées de muscles lisses et de leucocytes polymorphonucléaires de rat. Des travaux similaires effectués sur des préparations de membranes de tissus de poumon humain ont montré que ces tissus contiennent des récepteurs spécifiques du PAF [BIOCHEMICAL AND BIOPHYSICAL RESEARCH COMMUNICATIONS, Vol. 128, N° 2 (1985), pages 972-979].

Une protéine de fixation spécifique du PAF a été purifiée et caractérisée à partir de membranes de plaquettes humaines [VALONE, IMMUNOLOGY, 52 (1984), pages 169-174 ; NISHIHIRA et al., TOHOKU J. EXP. MED., 147 (1985), pages 145-152]. Toutefois, les rendements de purification sont faibles et la caractérisation de la molécule isolée en tant que récepteur spécifique du PAF reste à établir.

La présente invention s'est, en conséquence, donné pour but de pourvoir à une nouvelle préparation de récepteurs cellulaires du PAF qui présente une grande stabilité dans le temps et des propriétés de liaison avec le PAF améliorées, ainsi qu'à une nouvelle méthode de dosage du PAF à l'aide de récepteurs cellulaires, qui est plus sensible et plus spécifique que les méthodes de dosage immunologique et est plus spécifique que les méthodes de dosage biologique.

La présente invention a pour objet une préparation de récepteur du PAF qui est caractérisée par l'association d'une préparation membranaire de récepteur du PAF avec des agents de protection appropriés.

Selon un mode de réalisation de la préparation de récepteur du PAF conforme à la présente invention, les agents de protection susdits sont constitués par un système de stabilisation de la préparation de récepteur, qui comprend au moins un inhibiteur de protéases.

Selon une disposition avantageuse de ce mode de réalisation, ledit système de stabilisation comprend au moins un inhibiteur de pepsine et/ou au moins un inhibiteur de plasmine et/ou au moins un inhibiteur de trypsine et/ou au moins un inhibiteur de papaïne et/ou au moins un inhibiteur de cathepsine B et/ou au moins un inhibiteur de cathepsine D et/ou au moins un inhibiteur de chymotrypsine et/ou au moins un inhibiteur de sérine-protéinase.

Selon un autre mode de réalisation de la préparation de récepteur du PAF conforme à la présente invention, la préparation membranaire est constituée par des cellules entières ou des fragments de tissus, ou par des plaquettes purifiées isolées à partir de sang de mammifères appropriés, ou par des préparations résultant de la lyse desdites plaquettes, ou par des membranes de plaquettes purifiées, ou par des récepteurs membranaires solubilisés.

Selon encore un autre mode de réalisation de la préparation de récepteur du PAF conforme à la présente invention, celle-ci est constituée par une préparation stabilisée qui peut être soit lyophilisée, soit congelée.

La présente invention a, en outre, pour objet une méthode de dosage du PAF présent dans un échantillon biologique, dans laquelle on met en compétition un PAF marqué introduit dans le milieu de dosage, avec le PAF non marqué contenu dans l'échantillon biologique susdit, pour leur occupation des sites récepteurs du PAF présents dans une préparation de récepteur également introduite dans le milieu de dosage, caractérisé en ce que la préparation de récepteur du PAF susdite est constituée par l'association d'une préparation membranaire de récepteur du PAF avec des agents de protection et/ou de stabilisation appropriés.

Selon un mode de mise en oeuvre avantageux de la méthode de dosage conforme à l'invention, le PAF marqué utilisé dans la réaction de compétition susdite est un PAF marqué par un radioisotope ($^{125}I$, $^{14}C$, $^{3}H$,...) ou par une molécule chimique fluorescente, chimioluminescente, ou par une enzyme.

Selon un autre mode de mise en oeuvre avantageux de la méthode de dosage conforme à l'invention, la quantité de récepteur et la quantité de PAF marqué mises en oeuvre sont constantes, ce qui a pour effet qu'une augmentation du PAF non marqué dans le milieu de dosage réduit la quantité de PAF marqué qui se lie

au récepteur.

Selon encore un autre mode de mise en oeuvre de la méthode de dosage conforme à l'invention, le complexe molécule de PAF (marqué ou non) - récepteur à doser est séparé du PAF non lié au récepteur, par toute technique de séparation appropriée telle que séparation par précipitation, par centrifugation, par filtration sous vide notamment, et le signal porté par la molécule de PAF lié au récepteur, est révélé par tous moyens appropriés.

D'une manière connue, les moyens de révélation sont le comptage de la radioactivité, en compteur à scintillation, en cas de marquage radioactif, la révélation par un substrat approprié, pour un marquage par fluorescence ou par une enzyme, etc.

Selon un mode de mise en oeuvre de la méthode de dosage conforme à l'invention, préalablement à la réalisation du dosage, l'échantillon biologique contenant le PAF à doser est traité , si nécessaire, pour en extraire le PAF.

Selon un autre mode de mise en oeuvre de la méthode de dosage conforme à l'invention, le dosage est réalisé par établissement d'une courbe faisant apparaître le pourcentage de PAF qui se fixe au récepteur en présence d'un échantillon biologique contenant une quantité de PAF non marqué à doser, et comparaison de cette courbe avec une courbe standard de référence qui montre la décroissance du pourcentage de molécules de PAF marqué qui se fixe au récepteur en présence de quantités progressivement croissantes de PAF non marqué.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de préparation du récepteur du PAF conforme à la présente invention et de mise en oeuvre de la méthode de dosage qui fait également l'objet de la présente invention.

Il doit être bien entendu, toutefois, que ces exemples et les parties descriptives correspondantes sont donnés uniquement à titre d'illustration de l'objet de l'invention, dont ils ne constituent en aucune manière une limitation.

## EXEMPLES

### EXEMPLE 1 : OBTENTION D'UNE PREPARATION DE RECEPTEUR DU PAF.

1. du sang (environ 40 ml. ) prélevé chez un lapin est additionné de 8 ml de tampon ACD, qui est un tampon citrate dont la composition est la suivante :

| | |
|---|---|
| Citrate disodique, $2H_2O$ | $8,5.10^{-2}$ M |
| Acide citrique | $6,6.10^{-2}$ M |
| Glucose | $10^{-1}$ M |

Ce mélange est centrifugé à 37° C pendant 15 minutes à 250 g. Le culot est rejeté et le plasma riche en plaquettes (PRP) est centrifugé à 37° C, pendant 20 minutes à 600 g ; le surnageant est rejeté et le culot est recueilli.

2. Le culot est mis en suspension dans du tampon Tyrode, pH 7,4, dont la composition est la suivante :

| | | |
|---|---|---|
| Hepes | | $5\ 10^{-3}$ M |
| Glucose | | $5,5\ 10^{-3}$ M |
| BSA | | 0,35 % |
| NaCl | | 140 mM |
| KCl | | 2,6 mM |
| $NaHCO_3$ | | 12 mM |
| $NaH_2PO_4,H_2O$ | | 0,42 mM |
| $MgCl_2$ | | 1 mM |
| Apyrase | (SIGMA) | 0,3 UI/ml |
| EGTA | | 2 mM |
| Heparine | (ROCHE) | 50 UI/ml |

après centrifugation à 37° C, pendant 20 minutes à 600 g, le surnageant est écarté et le culot est recueilli,

puis traité dans du tampon TRIS-HCl-NaCl (pH 7,4), dont la composition est la suivante :

| | |
|---|---|
| Tris-HCl | 10 mM |
| NaCl | 150 mM |

3. La suspension obtenue est centrifugée à 37° C pendant 20 minutes à 600 g ; le surnageant est écarté et le culot est additionné de tampon TRIS-HCl, 10 mM, pH 7,0, et d'un système de stabilisation du récepteur du PAF dont la composition est la suivante :

| | | | |
|---|---|---|---|
| "PEPSTATINE" | (1) | (BOEHRINGER) | 0,0025 mg/ml |
| "ANTIPAINE" | (2) | " | 0,0025 mg/ml |
| "LEUPEPTINE" | (3) | " | 0,0025 mg/ml |
| "CHYMOSTATINE" | (4) | " | 0,0025 mg/ml |
| P M S F | (5) | (SERVA) | $2.10^{-4}$ M |

(1) inhibiteur de pepsine et de cathepsine D

(2) inhibiteur de papaïne et de trypsine

(3) inhibiteur de plasmine, de trypsine, de papaïne et de cathepsine B

(4) inhibiteur de chymotrypsine

(5) fluorure de phénylméthylsulfonyle inhibiteur de chymotrypsine, trypsine et protéases analogues.

4. On procède à la lyse des plaquettes par trois congélations/décongélations successives. Les plaquettes lysées sont centrifugées à + 4° C pendant 30 minutes à 50 000 g.
Le culot obtenu est repris par le tampon d'incubation et est congelé à - 80° C.

**EXEMPLE 2 : OBTENTION D'UNE PREPARATION DE RECEPTEUR DU PAF.**
Le matériel congelé à - 80° C obtenu en procédant comme décrit à l'Exemple 1 est lyophilisé.

**EXEMPLE 3 : VERIFICATION DES PROPRIETES DE LA PREPARATION DE RECEPTEUR DU PAF CONFORME A L'INVENTION;**
L'addition du système de protection et stabilisation décrit à l'Exemple 1 à la préparation membranaire de récepteur du PAF a un double effet :
- d'amélioration des qualités de liaison et
- d'amélioration de la durée de conservation.

3.1 AMELIORATION DES QUALITES DE LIAISON

3.1.1. Technique de Dosage :

Technique de Liaison

Matériel :
- 1 Aliquot de préparation membranaire de plaquettes de lapin (A)
- 1 Aliquot de préparation de récepteur du PAF conforme à l'invention (B),
- PAF marqué au Tritium, activité spécifique 56 Ci/mmole (DU PONT DE NEMOURS (Boston MA, USA).)
- PAF non marqué (BACHEM, Budendorf, Suisse).

Méthodes :
Des aliquots de préparations membranaires de plaquettes de lapin (~ 100 µg) sont incubés pour un volume final de 200 µl de solution contenant 1 nM de /$^3$H/PAF (15300 cpm) et soit une quantité de PAF non marqué connue (gamme de référence), soit un aliquot de dilution de l'extrait à doser. L'incubation est réalisée en tube

de polyéthylène à + 4° C pendant 1 heure 30, avec agitation constante (tampon d'incubation : TRIS-HCl 10 mM + BSA 0,025 %, pH 7,4). Chaque point du dosage est réalisé en triplicate.

Arrêt par filtration sous vide :
A la fin de la période d'incubation, 3 ml de tampon d'incubation à 4° C sont ajoutés au volume réactionnel et rapidement filtrés sur filtres Whatman GF/C (prétrempés en tampon d'incubation) par aspiration sous vide. Le tube est ensuite rincé 2 fois par 3 ml de tampon.

Détection du signal :
Les filtres sont placés dans des fioles de comptage avec 4 ml de liquide scintillant (Atomlight NEN). La radioactivité est quantifiée sur compteur à scintillation LKB Rack beta 1214.

Analyse des résultats :
La différence entre la quantité de $[^3H]$PAF lié aux membranes en absence et en présence d'un excès de PAF non marqué (1000 x $[^3H]$ PAF) est considérée comme liaison spécifique maximale ($B^*$). Les résultats sont analysés (logit) à partir de la liaison spécifique.

3.1.2. La Figure 1 annexée montre une augmentation significative du signal représentée par une augmentation de x 2,5 de la quantité de $[^3H]$ PAF lié spécifiquement à la préparation B conforme à l'invention par rapport à la quantité liée à la préparation A ne contenant pas de système de stabilisation.

3.2. Amélioration des délais de conservation :
Sans traitement, la qualité des préparapréparations de membranes de plaquettes n'est conservée que pour une durée ne dépassant pas 10 jours.

Le traitement par le système de stabilisation permet de maintenir les qualités des préparations de membranes sans altération jusqu'à un délai supérieur à un mois.

La lyophilisation des préparations de ré cepteur du PAF conformes à l'invention ne modifie pas les propriétés de liaison avec le /$^3H$ /PAF et les durées de conservation sont encore augmentées.

3.3. Spécificité :
Le site de liaison présent sur les préparations de membranes est spécifique pour la structure PAF. Plusieurs molécules, telles que le lyso-PAF (dérivé métabolique du PAF) ou divers phospholipides, ne sont pas reconnus par le site de liaison utilisé pour le dosage (dose maximale testée : $10^{-6}$M).

Cf. Figure 2 dans laquelle la spécificité est exprimée en valeurs normalisées ($B/B_o$) en fonction de la concentration de PAF (Gamme standard) ou de l'analogue testé (exprimé en nM).

**EXEMPLE 4 : DETERMINATION DE LA QUANTITE DE PAF PRESENTE DANS UN ECHANTILLON BIOLOGIQUE**

4.1. Caractéristiques du dosage :
Les caractéristiques du dosage ont été déterminées sur quatre expériences prises au hasard (indépendamment du lot de préparation et de la durée de conservation).
Sensibilité : déterminée par l'$ED_{50}$ (dose de PAF standard nécessaire pour inhiber 50 % de la liaison spécifique).
$ED_{50}$ = 5,73 x 1,38nM (0,63 + 0,15 ng/tube).
Limite de détection : : déterminée à partir du profil d'erreur représenté à la Figure 3 : le coefficient de variation a été déterminé sur quatre expériences prises au hasard.
Calcul du coefficient de variation :

$$\text{Coefficient de variation} = \frac{[X_T - \bar{X}] \times \sigma_{n-1}}{X_T}$$

$X_T$ dose théorique de PAF standard
X moyenne des valeurs lues de manière individuelle sur la courbe standard définie par la moyenne de 4 expériences.
$\sigma_{n-1}$ : écart type pondéré par n-1, calculé sur les valeurs déterminées individuellement.

Le coefficient de variation est exprimé en fonction du $B/B_o$ (donné en pourcent).

La partie de la courbe dont le coefficient de variation est inférieur ou égal à 10 %-15 % est utilisable pour le dosage.

Limite inférieure ($B/B_o$= 15 %) : 0,9 x 0,1 nM
(minimum détectable) (0,1 + 0,01 ng/tube)
Limite supérieure : 100 nM (11 ng/Tube)
(maximum détectable),
ces deux paramètres définissent l'ordre de grandeur dont il doit être tenu compte lors des dilutions des échantillons à doser.

4.2. La gamme standard est établie pour une série de concentrations de PAF standard réparties de façon équidistante en représentation logarithmique entre 0,1 nM et 1000 nM. Le pourcentage de [$^3$H ]PAF lié spécifiquement aux préparations en présence de ces différentes concentrations est déterminé par rapport à la quantité de [$^3$H ]PAF lié spécifiquement en absence de PAF standard non marqué ($B/B_o$ : B : cpm de [$^3$H ]PAF lié aux membranes en présence de PAF standard (liaison non spécifique déduite). Bo : cpm de [$^3$H ]PAF lié aux membranes en absence de PAF standard (liaison non spécifique déduite).

La liaison non spécifique est déterminée par un excès de PAF non marqué (1000 nM, soit un facteur 1000 par rapport à la quantité de [$^3$H ]PAF utilisé).

La Figure 4 annexée indique une gamme standard typique obtenue à partir des préparations de membranes de plaquettes de lapin.

Les valeurs de [$^3$H ]PAF sont reportées directement en cpm sur la gamme standard...
Sur cette courbe le Bo est de 9000 - 2900 = 6100 cpm
le non spécifique est de 2900 cpm.

Les déterminations de PAF des échantillons sont obtenues par interprétation sur la courbe standard.

La lecture des valeurs sur la représentation directe étant peu aisée, de par l'allure sigmoïde de la courbe, plusieurs transformations des résultats pouvant être utilisées pour linéariser la courbe. La transformation la plus utilisée est la transformation logit du pourcentage de reponse.

Sur la Figure 5 est représentée la transformation logit des résultats.

Description de la transformation logit :

Cette méthode de linéarisation est largement décrite dans la littérature ; elle est dérivée de la représentation de Hill.

Représentation de Hill

$$Y = \text{Log} \frac{B}{B_m - B} \quad \text{en fonction de Log F}$$

(Hill A.V. (1910)J Physiol 40 180)
B : Quantité de ligand liée spécifiquement pour une quantité totale de ligand libre F
$B_m$ : capacité maximale de liaison spécifique.

Représentation logit :

$$Y = \text{Log} \frac{B/B_o}{1 - B/B_o} \quad \text{en fonction de Log F}$$

B : quantité de ligand liée spécifiquement pour la dose F de ligand non marqué présente,
$B_o$ : quantité de ligand lié spécifiquement en absence de ligand non marqué.

Pour chaque point, il est soustrait la valeur de la liaison non spécifique déterminée par excès de PAF standard.

Le $B_o$ est déterminé par la quantité de [$^3$H ]PAF lié spécifiquement en absence de PAF standard :

$$\text{Logit : Log} \frac{B/B_o}{1 - B/B_o}$$

La détermination de la quantité x de PAF présente dans l'échantillon se fait par interpolation des pourcentages d'inhibition de la quantité x sur la liaison du [$^3$H ]PAF après transformation en logit.

4.3. Selon la nature de l'échantillon à doser, le PAF peut être dosé soit par dosage direct (si l'échantillon est

peu riche en éléments contaminants), soit par dosage après extraction.

L'échantillon doit être dilué ou concentré, suivant les cas, de manière à ce que les valeurs de PAF inconnues soient dans les limites du dosage (limite inférieure : 0,9 nM ; limite supérieure 100 nM).

Dans le cas où l'on choisit de doser après extraction, on extrait le PAF de l'échantillon par la méthode de BLIGH et DYER [CAN. J. BIOCHEM. PHYSIOL., 37 (1959), pages 911-918], en procédant comme suit :
- On arrête les réactions par addition au milieu biologique de méthanol et de chloroforme (1:1:1 v/v/v par rapport au volume de milieu biologique dont le PAF est à extraire).
- Agitation 10 minutes (Vortex),
- Séparation de phase amorcée par centrifugation (100 g, 5 minutes) ;
- Prélèvement de la phase organique,
- Lavages (2) par addition de chloroforme. (Le milieu biologique peut être, suivant les cas, soit un milieu de culture ou d'incubation, soit un broyat de tissus.)

Préparation de l'extraction pour le dosage :

Evaporation à sec de la phase organique, puis reprise par du tampon d'incubation (TRIS-HCl 10mM BSA 0,025 % pH 7,4). Réalisation de dilutions successives (entre $10^{-10}$M et $10^{-6}$M final dans le tube d'incubation) permettant le dosage du PAF contenu dans l'extrait.

Ainsi que cela ressort de ce qui précède, l'invention ne se limite nullement à ceux de ses modes de mise en oeuvre de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

**Revendications**

1°) Préparation de récepteur du PAF, caractérisée par l'association d'une préparation membranaire de récepteur du PAF avec des agents de protection appropriés.

2°) Préparation de récepteur du PAF, selon la Revendication 1, caractérisée en ce que les agents de protection susdits sont constitués par un système de stabilisation de la préparation de récepteur, qui comprend au moins un inhibiteur de protéases.

3°) Préparation de récepteur du PAF selon la Revendication 2, caractérisée en ce que ledit système de stabilisation comprend au moins un inhibiteur de pepsine et/ou au moins un inhibiteur de plasmine et/ou au moins un inhibiteur de trypsine et/ou au moins un inhibiteur de papaïne et/ou au moins un inhibiteur de cathepsine B et/ou au moins un inhibiteur de cathepsine D et/ou au moins un inhibiteur de chymotrypsine et/ou au moins un inhibiteur de sérine-protéinase.

4°) Préparation de récepteur du PAF selon l'une quelconque des Revendications 1 à 3, caractérisée en ce que la préparation membranaire est constituée par des cellules entières ou des fragments de tissus, ou par des plaquettes purifiées isolées à partir de sang de mammifères appropriés, ou par des préparations résultant de la lyse desdites plaquettes, ou par des membranes de plaquettes purifiées, ou par des récepteurs membranaires solubilisés.

5°) Préparation de récepteur du PAF selon l'une quelconque des Revendications 1 à 4, caractérisée en ce qu'elle est constituée par une préparation qui peut être soit lyophilisée, soit congelée.

6°) Méthode de dosage du PAF présent dans un échantillon biologique dans laquelle on met en compétition un PAF marqué introduit dans le milieu de dosage, avec le PAF non marqué contenu dans l'échantillon biologique susdit, pour leur occupation des sites récepteurs du PAF présents dans une préparation de récepteur également introduite dans le milieu de dosage, caractérisée en ce que la préparation de récepteur du PAF susdite est constituée par l'association d'une préparation membranaire de récepteur du PAF avec des agents de protection et/ou de stabilisation appropriés.

7°) Méthode de dosage selon la Revendication 6, caractérisée en ce que le PAF marqué utilisé dans la réaction de compétition susdite est un PAF marqué par un radioisotope approprié ($^{125}$I, $^{14}$C, $^{3}$H,..) ou par une molécule chimique fluorescente, chimioluminescente, ou par une enzyme, notamment.

8°) Méthode de dosage selon l'une quelconque des Revendications 6 ou 7, caractérisée en ce que la quantité de récepteur et la quantité de PAF marqué mises en oeuvre sont constantes, ce qui a pour effet qu'une augmentation du PAF non marqué dans le milieu de dosage réduit la quantité de PAF marqué qui se fixe au récepteur.

9°) Méthode de dosage selon l'une quelconque des Revendications 6 à 8, caractérisée en ce que le complexe molécule de PAF marqué ou non - récepteur, à doser, est séparé du PAF non lié au récepteur, par toute technique de séparation appropriée et le signal porté par la molécule de PAF lié au récepteur, est révélé par tous moyens appropriés.

10°) Méthode de dosage selon l'une quelconque des Revendications 6 à 9, caractérisée en ce que, préalablement à la réalisation du dosage, l'échantillon biologique contenant le PAF à doser est traité, pour en extraire le PAF.

11°) Méthode de dosage selon l'une quelconque des Revendications 6 à 10, caractérisée en ce que le dosage est réalisé par établissement d'une courbe faisant apparaître le pourcentage de PAF qui se fixe au récepteur en présence d'un échantillon biologique contenant une quantité de PAF non marqué à doser,

en comparaison de cette courbe avec une courbe standard de référence, qui montre la décroissance du pourcentage de molécules de PAF marqué qui se fixe au récepteur en présence de quantités progressivement croissantes de PAF non marqué.

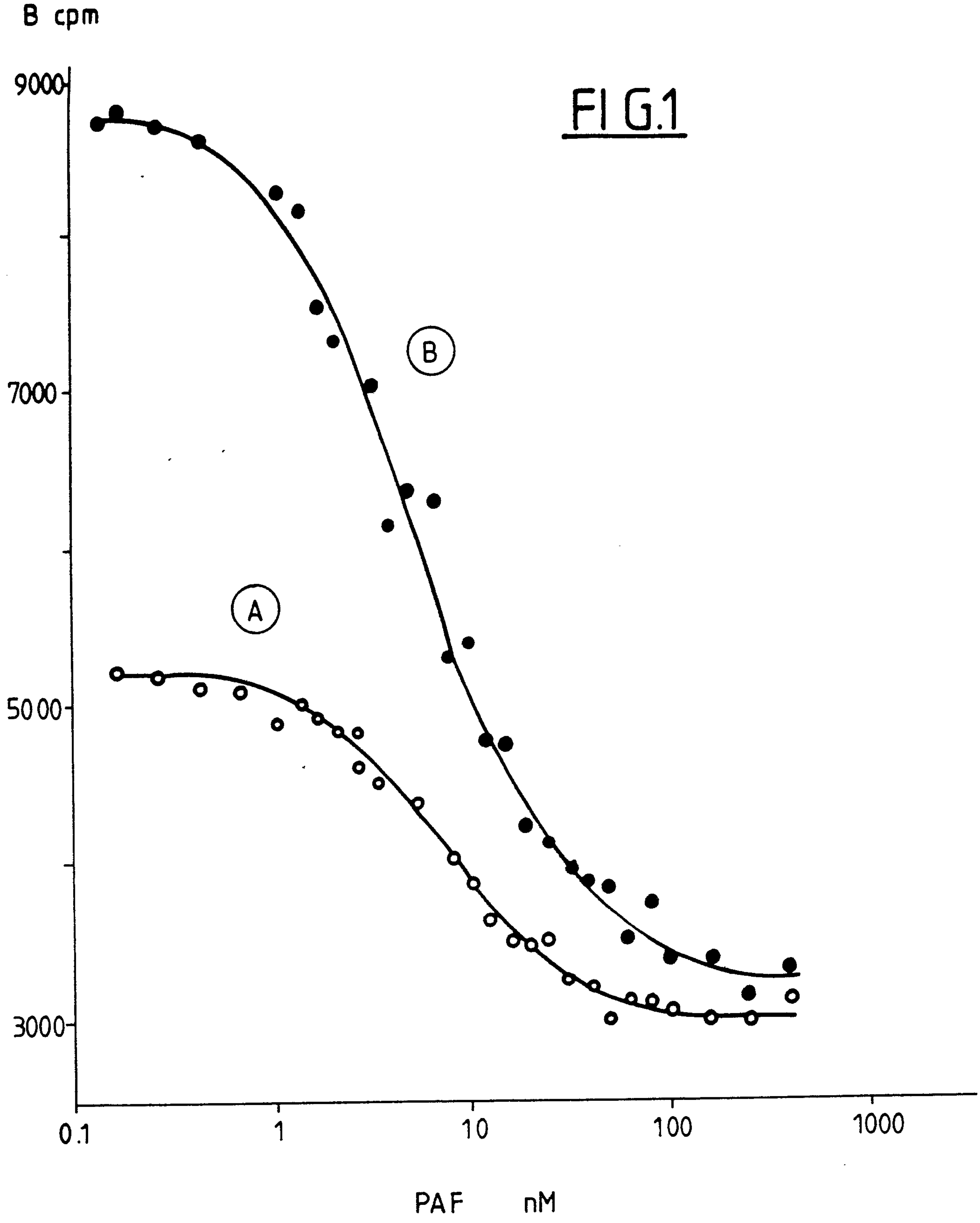


FIG.1

FIG. 2

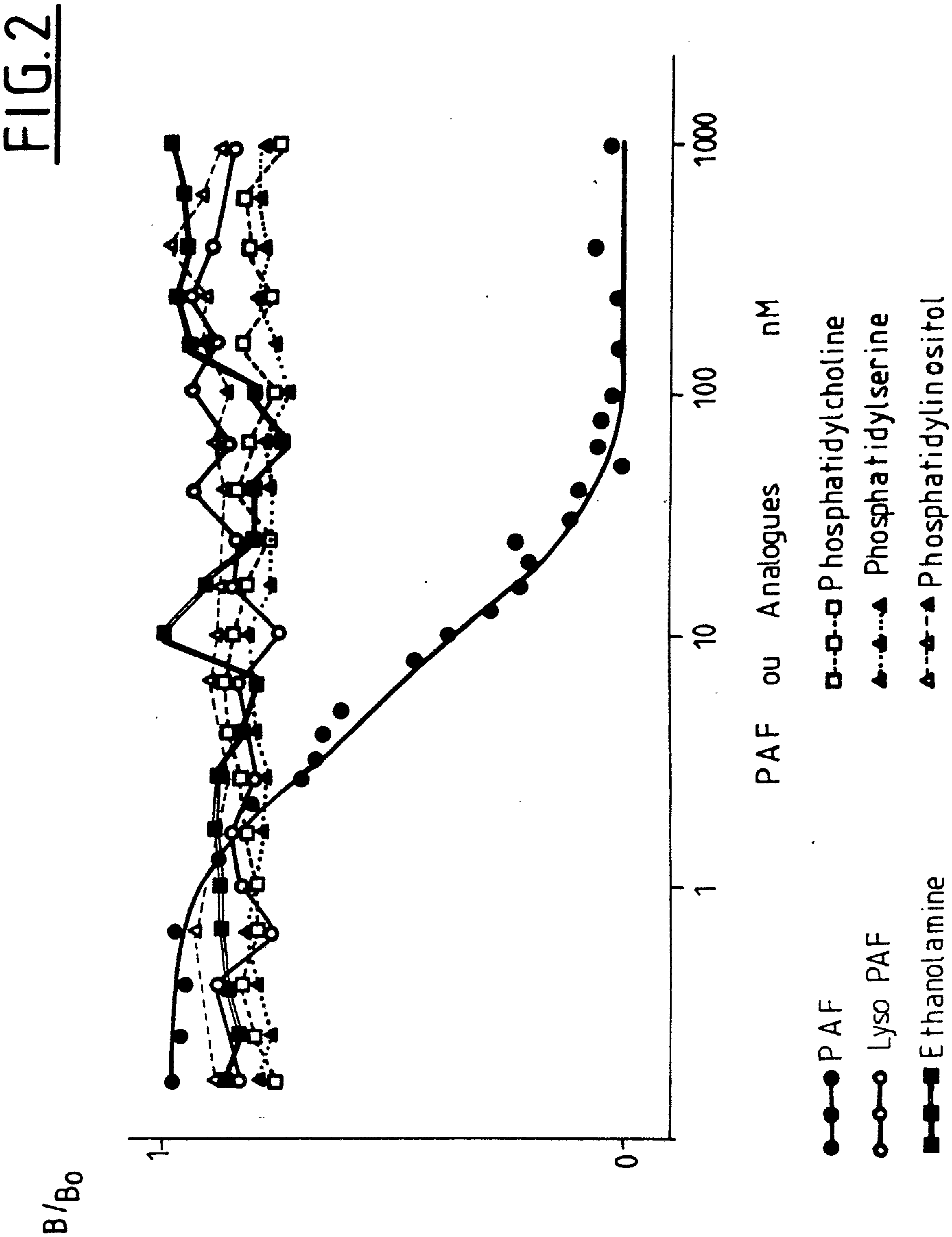
B/Bo
1
0
1
10
100
1000
PAF ou Analogues nM
PAF
Lyso PAF
Ethanolamine
Phosphatidylcholine
Phosphatidylserine
Phosphatidylinositol

FIG.3

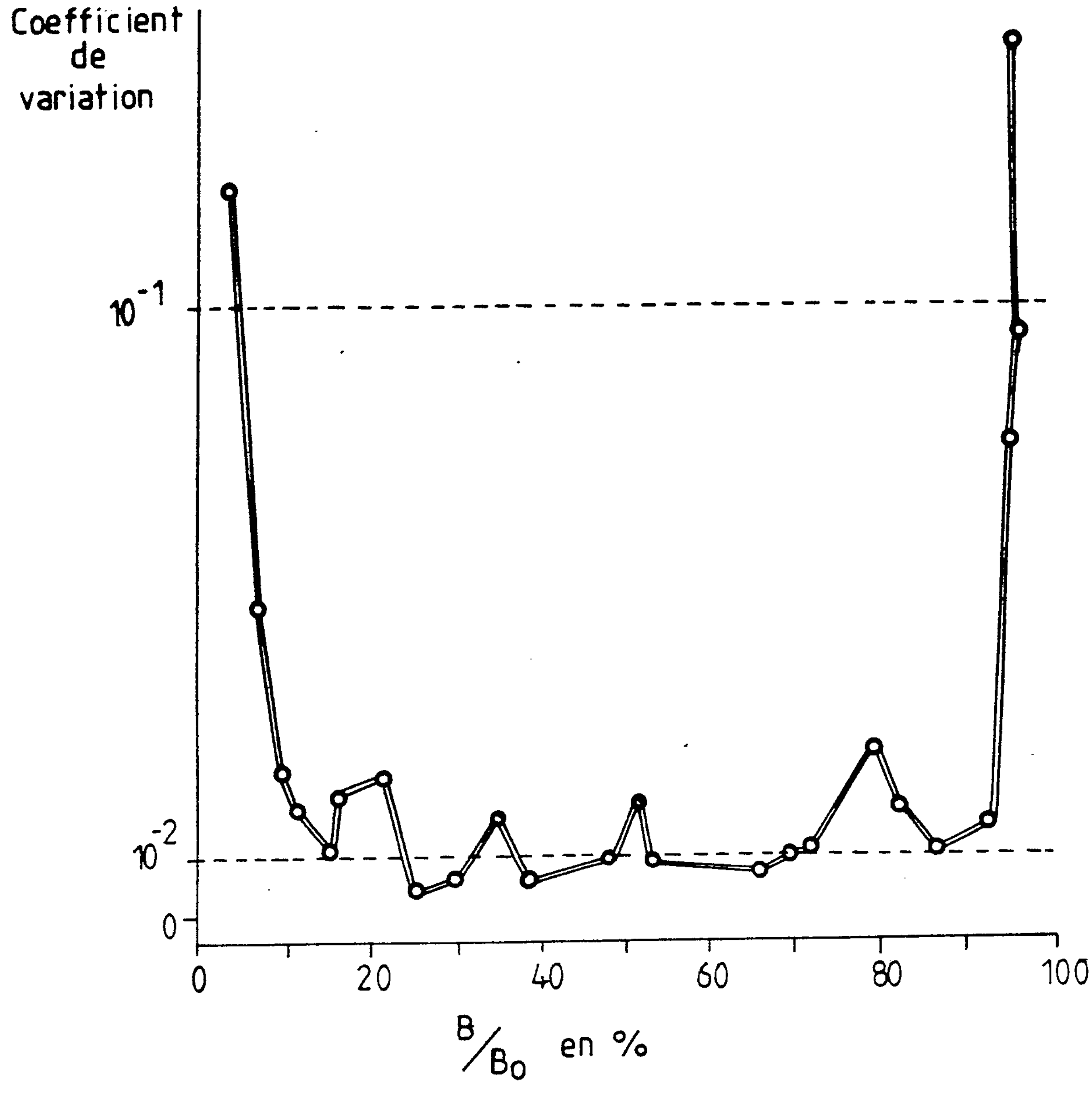

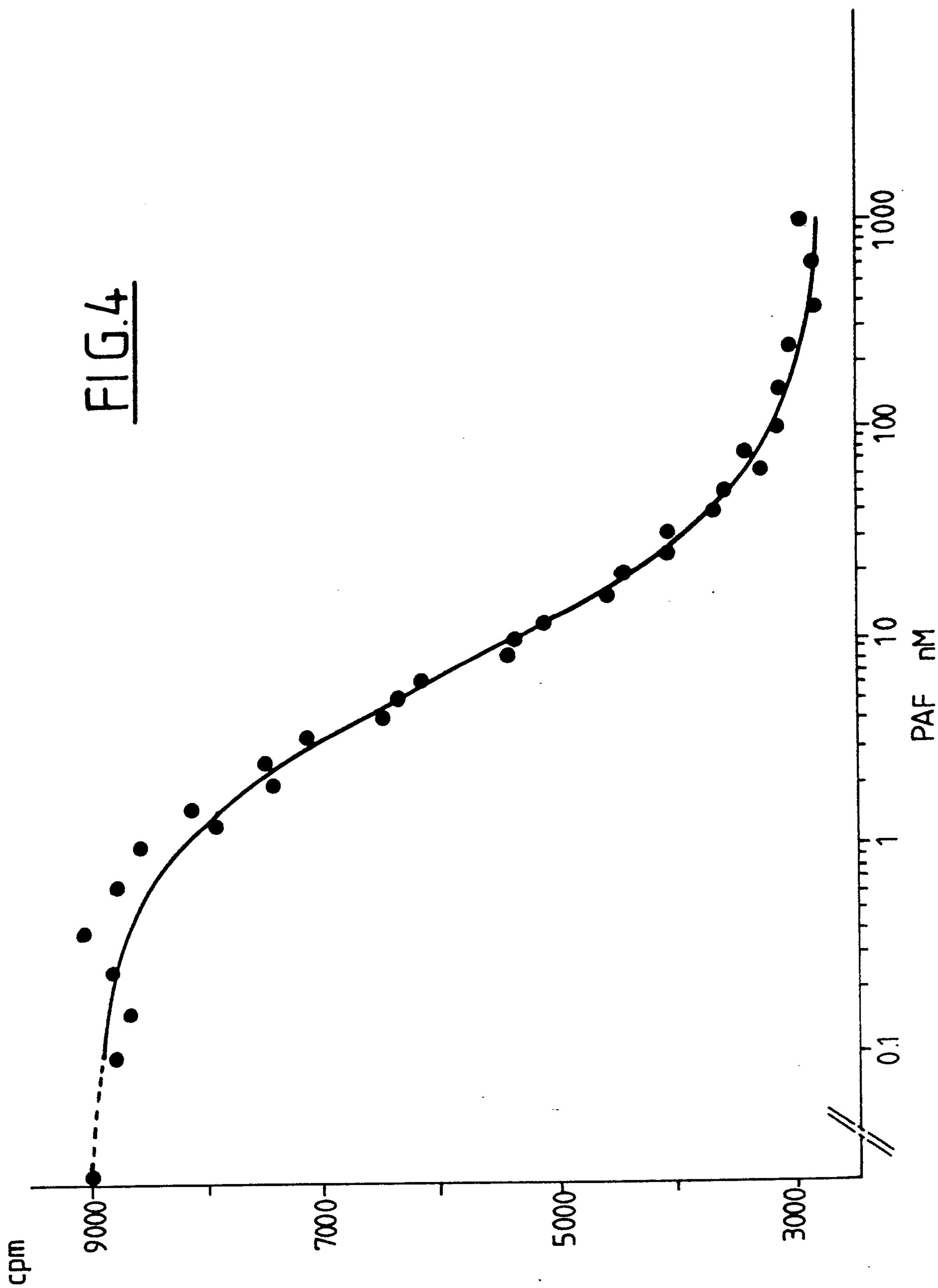


FIG.4

FIG.5

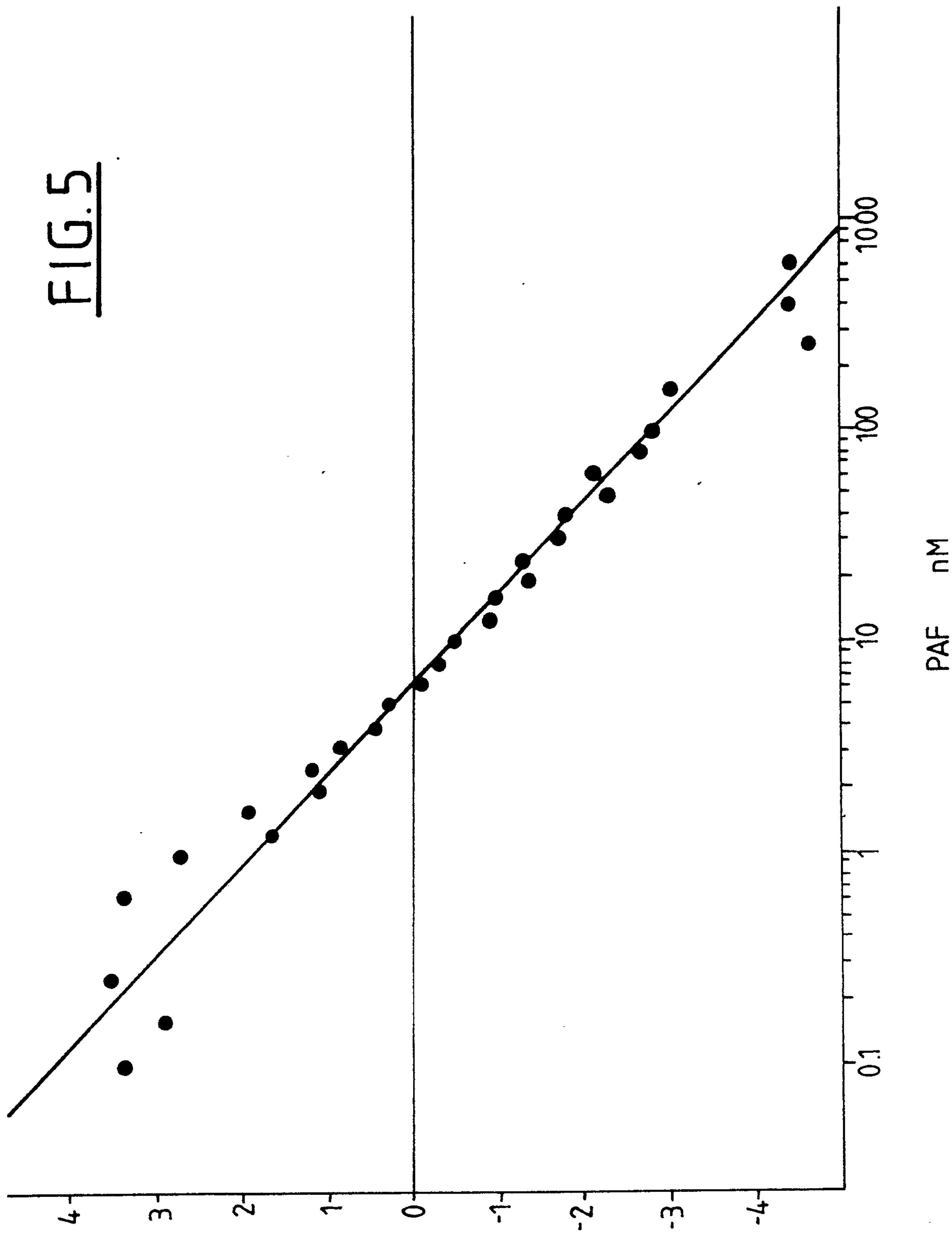

Office européen des brevets

# RAPPORT DE RECHERCHE EUROPEENNE

Numero de la demande

EP 89 40 1054

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | IMMUNOLOGY, vol. 52, 1984, pages 169-174; F.H. VALONE: "Isolation of a platelet membrane protein which binds the platelet-activating factor 1-O-hexadecyl-2-acetyl-SN-glycero-3-phosphorylcholine" * Résumé * --- | 1-5 | G 01 N 33/92 |
| Y | CHEMICAL ABSTRACTS, vol. 101, no. 1, 2 juillet 1984, page 282, no. 3217r, Columbus, Ohio, US; K. UMEZAWA et al.: "Elimination of protein degradation by use of protease inhibitors" * Résumé * --- | 1-5 | |
| A | FEDERATION PROCEEDINGS, vol. 41, no. 4, 1982, page 1459, no. 6950; T.F. MOWLES et al.: "Radioreceptor binding assay for platelet-activating factor (PAF)" * Résumé * --- | 1-12 | |
| | | | DOMAINES TECHNIQUES RECHERCHES (Int. Cl.4) |
| A | FEDERATION PROCEEDINGS, vol. 46, no. 3, 1 mars 1987, 71st Annual Meeting, Washington, DC, 29 mars - 2 avril 1987, page 740, no. 2475; S. PAULSON et al.: "Application of a radioreceptor binding assay for measurement of platelet activating factor synthesis by human neutrophils" * Résumé * --- -/- | 1-12 | A 61 K |

Le présent rapport a été établi pour toutes les revendications

| Lieu de la recherche | Date d'achèvement de la recherche | Examinateur |
|---|---|---|
| LA HAYE | 14-07-1989 | FERNANDEZ Y BRANAS F.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)